# EUROPÄISCHE PATENTANMELDUNG

(11) **EP 1 481 596 A1**
(43) Veröffentlichungstag der Anmeldung: **01.12.2004**
(21) Anmeldenummer: 03012005.9
(22) Anmeldetag: 28.05.2003
(51) Int. Cl.: A23L 1/30, A61K 9/50, A23P 1/04

(54) **Verwendung von Mikrokapseln**

(71) Anmelder: Cognis Iberia, S.L., 08755 Castellbisbal, (Barcelona) (ES)
(72) Erfinder: Viladot Petit, Josep-Lluis, Dr., 08018 Barcelona (ES)
(74) Vertreter: Fabry, Bernd, Dr.

(57) **Zusammenfassung**

Vorgeschlagen wird die Verwendung von Mikrokapseln mit mittleren Durchmessern im Bereich von 0,1 bis 500 µm, bestehend aus einer Hüllmembran und einer mindestens einen Wirkstoff enthaltenden Matrix, die dadurch erhältlich sind, dass man
(a) eine Lösung eines Wirkstoffes in einem geeigneten Lösungsmittel herstellt,
(b) die Lösung mit einem Polysaccharid versetzt,
(c) das Lösungsmittel von der Matrix abtrennt,
(d) den dabei erhaltenen Feststoff mit einer wässrigen Lösung eines kationischen Polymers versetzt,
(e) die Zubereitung auf einen pH-Wert größer oder gleich dem pKa-Wert des kationischen Polymers einstellt, und
(f) die Zubereitung mit den gebildeten Mikrokapseln anschließend entwässert,
als Nahrungsmittelergänzungsstoffe.

## Beschreibung

### Gebiet der Erfindung

Die Erfindung befindet sich auf dem Gebiet der Nahrungsmittelergänzungsstoffe und betrifft die Verwendung spezieller Mikrokapseln in diesem Gebiet sowie ein Verfahren zum Einschluss von in speziellen Wirkstoffen enthaltenen Geruchsträgern durch Mikroverkapselung der Stoffe.

### Stand der Technik

Im Gegensatz zum Nahrungsmittelsektor, der handelsübliche Produkte des täglichen Bedarfs umfasst, hat sich in den letzten Jahren ein davon zu unterscheidender Markt aufgetan, der Nahrungsmittelzusatzstoffe (auch als "functional food" bezeichnet) zum Gegenstand hat. Bei diesen Produkten handelt es sich im wesentlichen um hochkonzentrierte Wirkstoffe, die vom Konsumenten zusammen mit konventionellen Nahrungsmitteln oder separat davon, beispielsweise als Dragees, zu sich genommen werden, um damit einen physiologischen Effekt hervorzurufen. Das Spektrum der Wirkungen ist dabei sehr weit und reicht von der Stärkung der Abwehrkräfte über die Stimulation des Metabolismus bis hin zur Hautstraffung ("cosmetic inside"). Von Nachteil ist jedoch, dass die Wirkstoffe häufig über einen starken Eingeruch verfügen, der vom Verbraucher als nachteilig angesehen wird. In der Kosmetik kann dieses Problem oftmals durch Zugabe eines weiteren Duftstoffes gelöst werden, welcher den Geruch überdeckt oder maskiert, im Bereich der Nahrungsmittelergänzungsstoffe ist dies jedoch nicht möglich, zum einen da den meisten der für diesen Zweck in Betracht kommenden Duftstoffe die erforderliche Zulassung fehlt und zum anderen, da ein solcher Zusatz das Konzept eines möglichst reinen und natürlichen Produktes verwässern würde. Alternativ besteht die Möglichkeit, die Wirkstoffe zu verkapseln und somit die Geruchsträger mit einzuschließen. In der Praxis wurde jedoch beobachtet, dass übliche Verkapselungsverfahren zu wenig zufriedenstellenden Lösungen führen, da sich die Hüllsubstanzen nach kurzer Zeit doch für die Geruchsstoffe als durchlässig erwiesen.

In diesem Zusammenhang sei auf die europäische Patentanmeldung **EP 1101527 A1** (Cognis) verwiesen, aus der mikroverkapselte Wirkstoffe sowie deren Einsatz im Bereich der Nahrungsmittel bekannt sind. Die Matrix dieser Kapseln besteht im wesentlichen aus Stärke, während die Hülle von Chitosan gebildet wird.

Die Aufgabe der vorliegenden Erfindung hat folglich darin bestanden, einen Weg aufzuzeigen, auf dem sich Wirkstoffe mit starkem Eigengeruch so verkapseln lassen, dass auch bei längerer Lagerung keine Geruchsträger entweichen und die Bioverfügbarkeit der Wirkstoffe für die orale Aufnahme gewährleistet bleibt.

### Beschreibung der Erfindung

Gegenstand der Erfindung ist die Verwendung von Mikrokapseln mit mittleren Durchmessern im Bereich von 0,1 bis 500 µm, bestehend aus einer Hüllmembran und einer mindestens einen Wirkstoff enthaltenden Matrix, die dadurch erhältlich sind, dass man
(a) eine Lösung eines Wirkstoffes in einem geeigneten Lösungsmittel herstellt,
(b) die Lösung mit Polysacchariden versetzt,
(c) das Lösungsmittel von der Matrix abtrennt,
(d) den dabei erhaltenen Feststoff mit einer wässrigen Lösung eines kationischen Polymers versetzt,
(e) die Zubereitung auf einen pH-Wert größer oder gleich dem pKa-Wert des kationischen Polymers einstellt, und
(f) die Zubereitung mit den gebildeten Mikrokapseln anschließend entwässert,
als Nahrungsmittelergänzungsstoffe.

Überraschenderweise wurde gefunden, dass die Verkapselung von Wirkstoffen mit starkem Eigengeruch in einer Polysaccharid- bzw. Stärkematrix und einer Kationpolymerhülle, speziell einer Hülle aus Chitosan, im Hinblick auf den dauerhaften Einschluss der Geruchsträger besonders vorteilhaft ist, da andere Mikrokapseltypen sich für die geruchstragenden Stoffe schon nach kurzer Zeit als durchlässig erweisen. Die im Sinne der Erfindung zu verwendenden Kapseln zeigen sich jedoch auch nach monatelanger Lagerung noch vollkommen geruchsneutral.

Ein weiterer Gegenstand der Erfindung betrifft ein Verfahren zur olfaktorischen Neutralisation von Wirkstoffen mit starkem Eigengeruch, bei dem man
(a) eine Lösung eines Wirkstoffes in einem geeigneten Lösungsmittel herstellt,
(b) die Lösung mit Polysacchariden versetzt,
(c) das Lösungsmittel von der Matrix abtrennt,
(d) den dabei erhaltenen Feststoff mit einer wässrigen Lösung eines kationischen Polymers versetzt,
(e) die Zubereitung auf einen pH-Wert im Bereich von 6 bis 8 einstellt, und
(f) die Zubereitung mit den gebildeten Mikrokapseln anschließend entwässert.

### Wirkstoffe

Die Auswahl der im Sinne der Erfindung in Betracht kommenden Wirkstoffen ist wenig kritisch und richtet sich in erster Linie danach, welche Effekte erzielt werden sollen. Typische Beispiele sind Tocopherol, Tocopherolacetat, Tocopherolpalmitat, Ascorbinsäure, (Desoxy)Ribonucleinsäuren, Retinol, Bisabolol, Allantoin, Phytantriol, Panthenol, AHA-Säuren, Kojisäure, Lipoinsäure, Aminosäuren, Ceramiden, Pseudoceramide, essentielle Öle, Pflanzenextrakte und Vitaminkomplexe sowie deren Gemische.

### Polysaccharide

Als Polysaccharide eignen sich beispielsweise Stärke (Maisstärke, Weizenstärke, Reisstärke, Kartoffelstärke), Cellulose oder deren Derivate, insbesondere Carboxymethylcellulose.

### Kationpolymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1 , Mirapol® AZ-1 der Firma Miranol. Vorzugsweise werden als Kationpolymere Chitosane und darunter speziell solche Typen eingesetzt, die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Herstellung der Mikrokapseln

Zur Herstellung der erfindungsgemäßen Mikrokapseln empfiehlt es sich zunächst durch inniges Vermischen von Wirkstoffen und Polysacchariden eine wässrige Matrix herzustellen, in der die Wirkstoffe vorzugsweise unlöslich sind. Falls erforderlich, kann die Bildung der Matrix unter leichtem Wärmen erfolgen. Wie bei allen übrigen Verfahrensschritten auch ist es sehr empfehlenswert, die Zubereitungen intensiv zu rühren. Liegen die Wirkstoffe in fester Form vor, hat es sich als vorteilhaft erwiesen, sie zunächst in einem geeigneten Lösungsmittel, beispielsweise Ethanol oder Isopropylalkohol aufzunehmen und dann mit den Polysacchariden zu vermischen. Anschließend wird das Wasser bzw. das Lösungsmittel entfernt, wobei man die geformte Matrix dann als Feststoff, vorzugsweise als Pulver erhält. Werden flüssige Wirkstoffe eingesetzt, kann auf die Verwendung eines organischen Lösungsmittels jedoch verzichtet werden. Es ist dabei zu berücksichtigen, dass auch bei Einsatz von flüssigen Wirkstoffen nach der Trocknung feste Matrizes vorliegen, da eine besondere Eigenschaft der Polysaccharide und insbesondere der Stärke darin besteht, diese flüssigen Bestandteile zu adsorbieren. Das Einsatzverhältnis von Wirkstoff zu Polysaccharid ist wenig kritisch. Aus rein praktischen Gründen empfiehlt es sich jedoch innerhalb eines Gewichtsverhältnisses von 5 : 95 bis 95 : 5, vorzugsweise 10 : 90 bis 50 : 50 und insbesondere 15 : 85 bis 40 : 60 zu arbeiten. Nachdem die Matrix hergestellt worden ist, erfolgt die Zugabe der Kationpolymerlösungen; falls gewünscht kann man die Matrix auch schon zuvor in Wasser dispergieren. Für die Bildung der Hülle werden in der Regel verdünnte, beispielsweise 0,1 bis 1 Gew.-%ige wässrige Lösungen der Kationpolymere, im folgenden vorzugsweise Chitosansalze, vorzugsweise Chitosanglycolat verwendet. Obschon man die Chitosanlösung vorlegen und die Matrix hinzugeben kann, hat es sich als vorteilhafter erwiesen, umgekehrt zu verfahren und in die gebildete wässrige Matrix die Chitosanlösung einzutropfen. Das Gewichtsverhältnis Stärke zu Chitosan kann - bezogen auf den Feststoffgehalt der Chitosanlösung - 99 : 1 bis 50 : 50, vorzugsweise 95 : 5 bis 70 : 25 und insbesondere 90 : 10 bis 80 : 20 betragen. Die eigentliche Bildung der Hüllmembran erfolgt im letzten Schritt durch Zugabe von Basen, vorzugsweise 5 bis 25 Gew.%ige Alkalihydroxidlauge, wobei der pH-Wert mindestens des pKa-Wert des eingesetzten Kationpolymers erreichen muss. Wird Chitosan als kationisches Polymer verwendet, erfolgt die Einstellung des pH-Wertes auf einen Bereich von 6 bis 8, also in der Umgebung des Neutralpunktes. Anschließend werden die Zubereitungen entwässert, d.h. das Wasser entweder verdampft oder im Vakuum abgezogen, vorzugsweise wird aber gefriergetrocknet. Im Mittel zeigen die erhaltenen Mikrokapseln einen Durchmesser im Bereich von 0,1 bis 500, vorzugsweise 50 bis 100 µm sowie eine Wirkstoffbeladung von 1 bis 50, vorzugsweise 5 bis 25 und insbesondere 10 bis 20 Gew.-%. Die mit den Wirkstoffen beladenen Mikrokapseln können selbst in Mengen von 0,01 bis 10 und insbesondere 0,1 bis 1 Gew.-% - bezogen auf die Endprodukte - eingesetzt werden.

### Beispiele

### Beispiel 1

In einem Rührkolben wurde eine Mischung aus 1 g Valerianextrakt in 50 ml Wasser vorgelegt und mit 9 g Maisstärke versetzt. Nach intensivem Rühren wurde das Wasser im Vakuum abgezogen und der feste Rückstand mit 50 g einer 0,1 Gew.-%igen Lösung von Chitosanglycolat (Hydagen® CMF, Cognis) vermischt. Die Zubereitung wurde intensiv gerührt und dabei bis zum Neutralpunkt tropfenweise mit 15 Gew.-%iger Natriumhydroxidlösung versetzt. Anschließend wurde das Produkt gefriergetrocknet. Die Mikrokapseln besaßen einen mittleren Durchmesser von 75 µm.

### Beispiel 2

In einem Rührkolben wurde eine Mischung aus 1 g Rotkleeextrakt in 50 ml Wasser vorgelegt und mit 9 g Maisstärke versetzt. Nach intensivem Rühren wurde das Wasser im Vakuum abgezogen und der feste Rückstand mit 50 g einer 0,1 Gew.-%igen Lösung von Chitosanglycolat (Hydagen® CMF, Cognis) vermischt. Die Zubereitung wurde intensiv gerührt und dabei bis zum Neutralpunkt tropfenweise mit 15 Gew.-%iger Natriumhydroxidlösung versetzt. Anschließend wurde das Produkt gefriergetrocknet. Die Mikrokapseln besaßen einen mittleren Durchmesser von 70 µm.

### Beispiel 3

In einem Rührkolben wurde eine Mischung aus 1 g Lipoinsäure in 50 ml Wasser vorgelegt und mit 9 g Maisstärke versetzt. Nach intensivem Rühren wurde das Wasser im Vakuum abgezogen und der feste Rückstand mit 50 g einer 0,1 Gew.-%igen Lösung von Chitosanglycolat (Hydagen® CMF, Cognis) vermischt. Die Zubereitung wurde intensiv gerührt und dabei bis zum Neutralpunkt tropfenweise mit 15 Gew.-%iger Natriumhydroxidlösung versetzt. Anschließend wurde das Produkt gefriergetrocknet. Die Mikrokapseln besaßen einen mittleren Durchmesser von 80 µm.

## Patentansprüche

1. Verwendung von Mikrokapseln mit mittleren Durchmessern im Bereich von 0,1 bis 500 µm, bestehend aus einer Hüllmembran und einer mindestens einen Wirkstoff enthaltenden Matrix, dadurch erhältlich, dass man
(a) eine Lösung eines Wirkstoffes in einem geeigneten Lösungsmittel herstellt,
(b) die Lösung mit Polysacchariden versetzt,
(c) das Lösungsmittel von der Matrix abtrennt,
(d) den dabei erhaltenen Feststoff mit einer wässrigen Lösung eines kationischen Polymers versetzt,
(e) die Zubereitung auf einen pH-Wert größer oder gleich dem pKa-Wert des kationischen Polymers einstellt, und
(f) die Zubereitung mit den gebildeten Mikrokapseln anschließend entwässert,
als Nahrungsmittelergänzungsstoffe.

2. Verwendung nach Anspruch 1, **dadurch gekennzeichnet, dass** die Wirkstoffe ausgewählt sind aus der Gruppe, die gebildet wird von Tocopherol, Tocopherolacetat, Tocopherolpalmitat, Ascorbinsäure, (Desoxy)Ribonucleinsäuren, Retinol, Bisabolol, Allantoin, Phytantriol, Panthenol, AHA-Säuren, Kojisäure, Lipoinsäure, Aminosäuren, Ceramiden, Pseudoceramiden, essentiellen Ölen, Pflanzenextrakten und Vitaminkomplexen sowie deren Gemischen.

3. Verwendung nach den Ansprüchen 1 und/oder 2, **dadurch gekennzeichnet, dass** man die Mikrokapseln in Mengen von 0,01 bis 10 Gew.-% - bezogen auf die Zubereitungen - einsetzt.

4. Verfahren zur olfaktorischen Neutralisation von Wirkstoffen mit starkem Eigengeruch, **dadurch gekennzeichnet, dass** man
(a) eine Lösung der Wirkstoffe in einem geeigneten Lösungsmittel herstellt,
(b) die Lösung mit Stärke versetzt,
(c) das Lösungsmittel von der Matrix abtrennt,
(d) den dabei erhaltenen Feststoff mit einer wässrigen Lösung eines kationischen Polymers versetzt,
(e) die Zubereitung auf einen pH-Wert größer oder gleich dem pKa-Wert des kationischen Polymers einstellt, und
(f) die Zubereitung mit den gebildeten Mikrokapseln anschließend entwässert.
